# EUROPÄISCHE PATENTANMELDUNG

(11) **EP 2 846 606 A1**
(43) Veröffentlichungstag der Anmeldung: **11.03.2015**
(21) Anmeldenummer: 14183754.2
(22) Anmeldetag: 05.09.2014
(51) Int. Cl.: H05B 1/02, A61F 7/00, H05B 3/34

(54) **Elektrische Wärmvorrichtung**

(30) Priorität: 05.09.2013 DE 102013109731; 26.09.2013 DE 202013104385 U
(71) Anmelder: Leifheit AG, 56377 Nassau (DE)
(72) Erfinder: Stange, Pedro, 65582 Diez (DE); Moddick, Christian, 48317 Drensteinfurt (DE); Denk, Andre, 45481 Mülheim a.d.Ruhr (DE)
(74) Vertreter: Bungartz, Klaus Peter

(57) **Zusammenfassung**

Die Erfindung betrifft eine elektrische Wärmevorrichtung mit einem Wärme abgebenden Wärmeträger, der zumindest ein Elektroheizelement (2) aufweist, einer Einstellvorrichtung zur Einstellung der Temperatur an zumindest einer Oberfläche des Wärmeträgers in Abhängigkeit von einem einstellbaren Vorgabesignal, einer von einem Akkumulator gebildeten Energieversorgung, einer über eine Pulsweitenmodulation regel- oder steuerbaren Steuereinheit zum Zuführen der von der Energieversorgung bereitgestellten elektrischen Energie zu dem Elektroheizelement (2) als eine Folge von Energiepulsen mit einer vorgegebenen Frequenz und mit Pulsbreiten in Abhängigkeit von dem Vorgabesignal und der erfassten Temperatur, wobei das Elektroheizelement (2) die von der Energieversorgung bereitgestellte elektrische Energie in Wärmeenergie umzuwandeln vermag.

Die bekannten Wärmevorrichtungen werden mit hohen Frequenzen pulsweitenmoduliert gesteuert. Die kann bei mit hohen Stromstärken nahe am gesundheitsgefährdenden Bereich sein. Dies schließt die Erfindung mit höherer Sicherheit dadurch aus, dass die Frequenz der Energiepulse kleiner als 0,1 Hz, insbesondere gleich oder kleiner als 0,01 Hz ist.

## Beschreibung

Die Erfindung betrifft eine elektrische Wärmevorrichtung nach dem Oberbegriff des Anspruchs 1. Sie dient insbesondere als passive Wärmequelle zum Erwärmen eines menschlichen Körpers. Hierzu weist sie einem Wärme abgebenden Wärmeträger, mit zumindest einem Elektroheizelement, eine Einstellvorrichtung zur Einstellung der Temperatur an zumindest einer Oberfläche des Wärmeträgers in Abhängigkeit von einem einstellbaren Vorgabesignal, eine von einem Akkumulator gebildeten Energieversorgung, eine über eine Pulsweitenmodulation regel- oder steuerbaren Steuereinheit zum Zuführen der von der Energieversorgung bereitgestellten elektrischen Energie zu dem Elektroheizelement als eine Folge von Energiepulsen mit einer vorgegebenen Frequenz und mit Pulsbreiten in Abhängigkeit von dem Vorgabesignal und der erfassten Temperatur auf. Das Elektroheizelement vermag dabei die von der Energieversorgung bereitgestellte elektrische Energie in Wärmeenergie umzuwandeln.

Elektrische Wärmevorrichtungen sind allgemein bekannt. Sie werden als Wärmekissen oder Wärmedecken zum Wärmen von Patienten, als Sitzheizung, zum Beispiel zum Wärmen eines Autositzes oder auch als Wärmplatte, zum Beispiel zum Warmhalten von Speisen eingesetzt.

Elektrische Wärmevorrichtungen nutzen unterschiedliche Energiequellen wie z.B. die Netzspannung oder einen Akku. In der Regel haben diese Wärmevorrichtungen eine oder mehrere einstellbare Temperaturstufen.

Eine einfache und nahezu verlustfreie Möglichkeit unterschiedliche Leistungen für die verschiedenen Temperaturstufen einzustellen, ist die Nutzung einer Pulsweitenmodulation (pulse width modulation, PWM). Zu Beginn des Aufwärmvorgangs wird in der Regel die volle von der Energiequelle zur Verfügung gestellte Leistung genutzt, um eine möglichst schnelle Erwärmung zu gewährleisten. Anschließend wird für die verschiedenen Temperaturstufen die Leistung durch eine Pulsweitenmodulation mit unterschiedlichen Taktraten (Duty-cycles) geregelt.

Der Stand der Technik weist jedoch einige Nachteile auf. Insbesondere aufgrund der niedrigen Spannung von Akkusystemen im Vergleich zur Netzspannung sind relativ hohe Ströme notwendig, um die erforderliche Leistung für die gewünschten Temperaturen zu erzielen. Für eine möglichst stetige Leistungsregelung wird vorzugsweise eine hochfrequente Pulsweitenmodulation im Bereich von einigen Hertz (Hz) bis zu einigen Kilohertz (kHz) genutzt.

Durch die Verwendung der hochfrequenten Pulsweitenmodulation entstehen starke elektromagnetische Felder, die möglicherweise nicht unkritisch sind, insbesondere besteht die Gefahr, dass ohne konkrete gesundheitliche Gefahren solche Felder in Zeiten einer zunehmenden Kritik der Verbraucher an elektromagnetischen Feldern nicht mehr toleriert werden und zu einer Kritik am Produkt führen können. Ferner könnte es sein, auch wenn dies von Experten als eher unwahrscheinlich angenommen wird, dass hochfrequente elektrische Wechselfelder doch gesundheitsschädlich zu sein könnten, so zum Beispiel Tumorerkrankungen begünstigen könnten.

Es wäre daher wünschenswert, die für die Wärmeerzeugung zugeführte Leistung regeln zu können, ohne dafür hochfrequente Pulsweitenmodulation einsetzen zu müssen. Damit ließen sich Wärmevorrichtungen herstellen, die die oben genannten Probleme von vorneherein ausschließen würden.

Dies ist umso erstrebenswerter, als Wärmevorrichtungen in manchen Anwendungen wohltuend und entspannend wirken und gegen Verspannungen eingesetzt werden, wobei sie sich in unmittelbarer Nähe zum menschlichen Körper befinden.

Aufgabe der Erfindung ist es daher, eine Wärmevorrichtung zu schaffen, bei der die zugeführte elektrische Leistung ohne Erzeugung potentiell störender hochfrequenter Strahlung geregelt wird.

Diese Aufgabe wird durch die elektrische Wärmevorrichtung gelöst, bei der die Frequenz der Energiepulse in Abhängigkeit von der Oberfläche und der Wärmekapazität des Wärmeträgers vorgegeben wird. Bevorzugte Ausführungsformen der Erfindung sind Gegenstand der Unteransprüche.

Der Erfindung liegen Untersuchungen zugrunde, die gezeigt haben, dass in Abhängigkeit der Oberfläche und der Wärmekapazität des Wärmeproduktes, d.h. des Wärmeträgers auch eine deutlich niederfrequentere Pulsweitenmodulation zur Leistungsregelung genutzt werden kann. Bei entsprechender Auslegung dieser Parameter sind spürbare Temperaturänderungen aufgrund der Trägheit des Wärmeübertragungssystems an der Oberfläche für den Nutzer kaum oder nicht wahrnehmbar. Selbst bei Unterbrechungen des Heizstromes von mehreren Sekunden ist während der Anwendung kein Unterschied im Vergleich zu einer hochfrequenten Regelung feststellbar.

Der menschliche Körper ist durch die Verwendung der niederfrequenten Pulsweitenmodulation nicht mehr den starken und sich schnell ändernden elektromagnetischen Feldern ausgesetzt. Ferner kann die Störung anderer elektrischer Geräte deutlich reduziert werden. Man kann eher von statischen Feldern sprechen, die nach heutigem Wissensstand nicht gesundheitsschädlich sind.

Als weitere Möglichkeit kann zur Vermeidung starker elektromagnetischer Wechselfelder ein System mit mehreren Heizdrähten unterschiedlichen Widerstands eingesetzt werden. Je nach Anzahl n der Heizdrähte können 2ⁿ - 1 verschiedene Leistungsstufen implementiert werden.

Auf eine PWM kann dann komplett verzichtet werden, und jede Stufe wird mit einer konstanten Leistung betrieben.

Die gattungsgemäße elektrische Wärmevorrichtung zum Einstellen einer Temperatur an einer Oberfläche eines Wärmeträgers in Abhängigkeit von einem Vorgabesignal ist versehen mit einem Temperatursensor zum Erfassen der Temperatur an der Oberfläche des Wärmeträgers, wenigstens einem Elektroheizelement zum Wandeln elektrischer Energie in Wärmeenergie und einer Steuereinheit zum Zuführen der elektrischen Energie zu dem wenigstens einen Elektroheizelement als eine Folge von Energiepulsen mit einer vorgegebenen Frequenz und mit Pulsbreiten in Abhängigkeit von dem Vorgabesignal und der erfassten Temperatur.

Erfindungsgemäß wird die elektrische Wärmevorrichtung dadurch weitergebildet, dass die Frequenz der Energiepulse in Abhängigkeit von der Temperatur der Oberfläche und der Wärmekapazität des Wärmeträgers vorgegeben wird. Die vorgegebene Frequenz der Energiepulse ist kleiner als 0,1 Hz und insbesondere kleiner als 0,01 Hz. Bei derartig niedrigen Werten für die Schaltfrequenz ist die Erzeugung von unerwünschter hochfrequenter Strahlung sehr gering.

Die Erfindung macht sich dabei die Tatsache zunutze, dass die Trägheit der Wärmeleitung innerhalb der Wärmevorrichtung dafür Sorge trägt, dass die niederfrequentierte Pulsweitenmodulation vom Benutzer nicht bemerkt wird. Eine Möglichkeit, diese niederfrequente Pulsweitenmodulation noch mehr zu kompensieren besteht entweder in der Verwendung eines passiven Wärmespeichers, der die Trägheit der Wärmevorrichtung noch weiter erhöht, oder in der Verwendung mehrerer Elektroheizelemente, die parallel zueinander angeordnet sein können und Phasen verschoben mit Energie versorgt werden.

So kann beispielsweise als Elektroheizelement ein Heizleiter gebildet werden, der drei einzelne Heizleiter aufweist, die entweder parallel zueinander nebeneinanderliegen oder miteinander verdreht sein können. Zur Vermeidung eines elektrischen Kontaktes der Heizleiter untereinander sind die einzelnen Heizleiter elektrisch isoliert. Jeder Heizleiter kann dann um 120° Phasen verschoben relativ zu einem anderen Heizleiter mit Energie versorgt werden, so dass sich keine Phasen ergeben, in denen keiner der Heizleiter aktiv beschickt wird.

Die erfindungsgemäße Wärmevorrichtung kann, insbesondere dann, wenn durch die Verwendung der niederfrequenten Pulsweitenmodulation doch eine Wärmeschwankung auftreten sollte, mehrere Elektroheizelemente umfassen. Diese sind dann bevorzugt parallel oder fast parallel zueinander angeordnet und können zum Beispiel dadurch erwärmt werden, dass an den Elektroheizelementen die Energiepulse von der Steuereinheit oder der Regelung zur Einstellung der Heizleistung mit unterschiedlicher Phase angelegt werden. Auf diese Art lässt sich eine gleichmäßigere Wärmeerzeugung erreichen.

Um ein bestimmtes Wärmeprofil auf der Oberfläche des Wärmeträgers zu erzielen, können bei Verwendung mehrerer Elektroheizelemente diese jeweils einen unterschiedlichen Widerstand aufweisen. Ferner können natürlich bei Verwendung von mehreren Heizelementen diese auch mit unterschiedlichen Pulsweiten geregelt werden.

Vorzugsweise werden die n Elektroheizelemente so angesteuert, dass 2ⁿ - 1 verschiedene Leistungsstufen implementiert sind.

Um die durch die steilen Flanken der Energiepulse erzeugten hochfrequenten Strahlungsanteile zu unterdrücken, werden die Energiepulse bei einer bevorzugten Ausführungsform der Erfindung durch ein Tiefpassfilter gefiltert. Auf diese Art werden die Flanken abgeflacht und Spannungsänderungen abgerundet, so dass hochfrequente Strahlung gar nicht erst entsteht.

Eine weitere vorteilhafte Ausgestaltung der Erfindung schirmt auf einer Seite durch eine thermische Isolation das Elektroheizelement ab. Auf der gegenüberliegenden Seite oder nahe dem Elektroheizelement kann dann ein passiver Wärmespeicher als Puffer vorgesehen sein, der die abgegebene Wärmemenge harmonisiert und so die durch die niederfrequentierte Leistungsversorgung entstehenden Temperaturschwankungen glättet. Natürlich kann der Wärmespeicher auch von der Oberfläche selbst gebildet sein.

Als passiver Wärmespeicher kommen alle denkbaren Materialien und Formen in Betracht, die innerhalb der Wärmevorrichtung angeordnet werden können und Wärme aufnehmen sowie kontinuierlich wieder abgeben können. Hier kommen natürlich bevorzugt metallische Gewebe oder Gelkissen bzw. -lagen in Betracht. Je nach Ausgestaltung dieses Wärmespeichers kann dies dazu führen, dass die Wärmevorrichtung auch als Kühlvorrichtung benutzt werden kann. Hierzu kann dann die Wärmevorrichtung zum Beispiel in den Kühlschrank oder Gefrierschrank eingelegt werden und dort heruntergekühlt werden. So kann beispielsweise eine Heizdecke im Sommer als kühlende Unterlage verwendet werden.

Die Erfindung ist besonders in Verbindung mit akkubetriebenen Wärmevorrichtungen nützlich, da hier hohe Stromstärken und damit große elektrische Felder auftreten.

Weitere Merkmale und Vorteile der Erfindung ergeben sich aus den Unteransprüchen und einer Beschreibung eines bevorzugten Ausführungsbeispiels anhand der Zeichnungen.

In den Zeichnungen zeigt:
- Fig. 1:: eine erste Ausgestaltung eines Wärmekissens nach der Erfindung in einer Seitenansicht und
- Fig. 2:: eine alternative Ausgestaltung eines Wärmekissens in einer Schnittansicht.

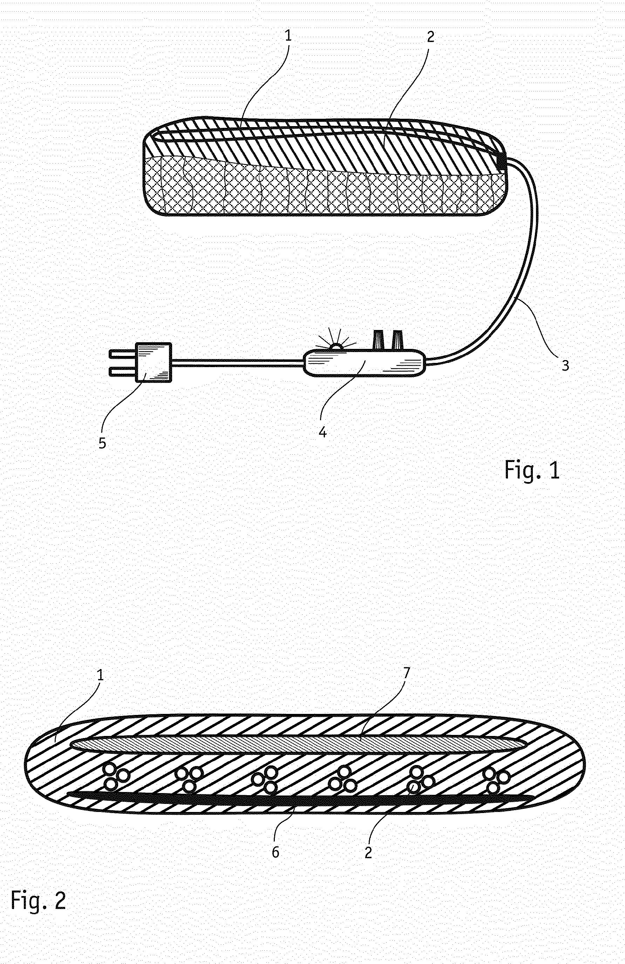

In Figur 1 ist eine als Wärmekissen 1 ausgebildete elektrische Wärmevorrichtung dargestellt, die über einen Stecker 5 und ein Anschlusskabel 3 mit der Netzspannung verbunden werden kann. Im Anschlusskabel 3 ist eine Einstellvorrichtung 4 vorgesehen, über die der Benutzer die Temperatur an der Oberfläche der elektrischen Wärmevorrichtung einstellen kann.

Im gezeigten Ausführungsbeispiel weist das Wärmekissen 1 als Elektroheizelement 2 einen elektrischen Leiter auf, der schleifenartig mit einem Hin- und einem Rückleiter an der Oberfläche angeordnet ist. Dieser Heizleiter wird über eine Steuerung mit Leistung beschickt, wobei die Steuerung zur Regulierung der Leistung und damit der Temperatur eine Pulsweitenmodulation aufweist. Dies bedeutet, dass die Länge der einzelnen Pulse und bzw. oder der Abstand der Pulse zueinander moduliert wird. Erfindungsgemäß wird nun eine niederfrequente Pulsweitenmodulation eingesetzt.

Da die Wärmeleitung der verwendeten Materialien vergleichsweise träge ist, bemerkt der Benutzer die relativ langen Pulse bzw. Pausen zwischen einzelnen Pulsen so gut wie nicht. Dafür hat die Herabsetzung der Frequenz der Pulsweitenmodulation den besonderen Vorteil, dass die Einflüsse durch das hierdurch entstehende elektrische Feld deutlich minimiert sind, so dass gesundheitliche Gefahren erheblich reduziert sind.

Gegebenenfalls kann das Elektroheizelement 2 nochmals mit einem Ausgleichselement oder einer Ausgleichsschicht ummantelt werden, die eine relativ hohe Wärmekapazität aufweist, so dass zusätzlich die Einflüsse durch die schwankende Leistung reduziert werden können. Diese Ausgleichsschicht kann mantelartig unmittelbar um die Elektroheizelemente 2 herumgelegt werden oder auch nur als Zwischenschicht in der elektrischen Wärmevorrichtung vorgesehen sein und beispielsweise ein wärmespeicherndes Gel enthalten oder aus Drahtgewebe gefertigt sein, wobei auch alle anderen Materialien verwendet werden können, die eine hinreichende Wärmekapazität aufweisen.

Die Ausgleichsschicht oder das Ausgleichselement weisen eine hohe Wärmekapazität auf, sodass ein passiver Wärmespeicher 7 hierdurch geschaffen werden kann. Je nach Ausgestaltung der Wärmevorrichtung kann dieser Wärmespeicher 7 unterschiedlich ausgebildet sein.

Ist die Wärmevorrichtung beispielsweise als Kissen ausgebildet, kann in diesem Kissen zusätzlich zu dem Elektroheizelementen 2 bzw. dem Elektroheizelement 2 ein metallisches Gewebe oder eine Folie eingelegt sein. Alternativ kann auch ein Gelkissen verwendet werden, dass eine hohe Wärmekapazität aufweist. Neben der Tatsache, dass dieser Wärmespeicher die durch die geringe Frequenz der niederfrequenten Pulsweitenmodulation entstehenden Wärmeschwankungen ausgleicht kann er darüber hinaus die Abkühlphase nach in Fall der elektrischen Energie harmonischer gestalten und verlängern sowie bei einer besonderen Ausgestaltung sogar eine doppelte Möglichkeit für die Beschickung der Wärmevorrichtung mit Energie bieten. Sofern die Wärmekapazität groß genug ist kann es dann nämlich ausreichend sein, den Wärmespeicher 7 über eine anderweitige Wärmequelle, beispielsweise den Backofen zu erwärmen.

Alternativ kann der Wärmespeicher auch in Form eines metallischen Geflechts rund um das dann als Heizdraht ausgebildete Elektroheizelement 2 herum angeordnet sein. Sofern mehrere Elektroheizelemente 2 verwendet werden, die als Bündel zusammengefasst sind, kann diese Ummantelung durch den Wärmespeicher 7 das gesamte Bündel umschließen oder jeden einzelnen Heizdraht.

Figur 2 zeigt einen möglichen Aufbau eines Wärmekissens in einer Schnittansicht. Hier ist ein Hin und herlaufendes Elektroheizelement 2 in Form eines Bündels von drei Heizdrähten vorgesehen. Unterhalb des Elektroheizelementes 2 ist eine thermische Isolation 6 angeordnet, die ein Abstrahlen der Wärme in die dem Benutzer abgewandte Richtung unterdrückt. Die Oberfläche des Wärmekissens kann entsprechend gestaltet sein, beispielsweise über Farbgebung und oder andere optische Hinweise, sodass der Benutzer gut erkennen kann, welche Seite die aktive Seite ist. Dies hat zusätzlich den Vorteil, dass der Benutzer dann, wenn er aus Versehen eine zu große Wärme eingestellt hat, das Kissen wenden und die kühlere Seite benutzen kann.

Oberhalb des Elektroheizelementes 2 ist ein Wärmespeicher 7, hier in Form eines Kissens angeordnet. Dieses Kissen ist mit einem Gel gefüllt, dass thermische Energie speichern kann. Damit der Benutzer nach Einschalten der Wärmevorrichtung nicht zu lange warten muss, kann die Steuerung so ausgelegt sein, dass sie zunächst mit maximaler Heizleistung anheizt, bis der Wärmespeicher 7 aufgewärmt ist. Dies ist besonders praktisch zu realisieren, wenn das Heizkissen einen Temperatursensor aufweist, so dass die Steuerung die Heizleistung dann zurücknehmen kann, wenn die thermische Energie durch das den Wärmespeicher 7 bildende Gelkissen hindurch zur Oberfläche gelangt.

### Bezugszeichenliste:

- 1: Wärmekissen
- 2: Elektroheizelement
- 3: Anschlusskabel
- 4: Einstellvorrichtung
- 5: Stecker
- 6: Thermische Isolation
- 7: Wärmespeicher

## Patentansprüche

1. Elektrische Wärmevorrichtung mit
• einem Wärme abgebenden Wärmeträger, der zumindest ein Elektroheizelement (2) aufweist,
• einer Einstellvorrichtung zur Einstellung der Temperatur an zumindest einer Oberfläche des Wärmeträgers in Abhängigkeit von einem einstellbaren Vorgabesignal,
• einer von einem Akkumulator gebildeten Energieversorgung,
• einer über eine Pulsweitenmodulation regel- oder steuerbaren Steuereinheit zum Zuführen der von der Energieversorgung bereitgestellten elektrischen Energie zu dem Elektroheizelement (2) als eine Folge von Energiepulsen mit einer vorgegebenen Frequenz und mit Pulsbreiten in Abhängigkeit von dem Vorgabesignal und der erfassten Temperatur,
wobei das Elektroheizelement (2) die von der Energieversorgung bereitgestellte elektrische Energie in Wärmeenergie umzuwandeln vermag,
**dadurch gekennzeichnet, dass**
die Frequenz der Energiepulse kleiner als 0,1 Hz, insbesondere gleich oder kleiner als 0,01 Hz ist.

2. Elektrische Wärmevorrichtung nach Anspruch 1, **dadurch gekennzeichnet, dass** die elektrische Wärmevorrichtung einen Temperatursensor zum Erfassen der Temperatur an der Oberfläche des Wärmeträgers und eine Regelung zur Einstellung der Heizleistung aufweist, wobei die Steuereinheit in Abhängigkeit der gemessenen Temperatur und einer vom Benutzer über eine Einstellvorrichtung (3) vorgegebenen Wunschtemperatur die Pulsweite der Energieimpulse moduliert.

3. Elektrische Wärmevorrichtung nach einem der beiden vorhergehenden Ansprüche, **dadurch gekennzeichnet, dass** die elektrische Wärmevorrichtung als Wärmekissen (1), als Wärmedecke, als Sitzheizung oder als Wärmplatte zum Warmhalten von Speisen ausgebildet ist.

4. Elektrische Wärmevorrichtung nach einem der vorhergehenden Ansprüche, **dadurch gekennzeichnet, dass** das Elektroheizelement (2) ein Heizdraht ist.

5. Elektrische Wärmevorrichtung nach einem der vorhergehenden Ansprüche, **dadurch gekennzeichnet, dass** die elektrische Wärmevorrichtung zwei oder mehr Elektroheizelemente (2) aufweist, wobei die Energieversorgung die Energiepulse phasenverschoben den Elektroheizelementen (2) bereitzustellen vermag, wobei die Phasen um 360° dividiert durch die Anzahl der Elektroheizelemente (2) verschoben sind.

6. Elektrische Wärmevorrichtung nach dem vorhergehenden Anspruch, **dadurch gekennzeichnet, dass** die Elektroheizelemente (2) einem Bündel zusammengefasste und elektrisch voneinander isoliert Heizdrähte sind.

7. Elektrische Wärmevorrichtung nach Anspruch 6, **dadurch gekennzeichnet, dass** die Elektroheizelemente (2) jeweils einen unterschiedlichen elektrischen Widerstand aufweisen.

8. Elektrische Wärmevorrichtung nach einem der Anspruch 6, **dadurch gekennzeichnet, dass** die elektrische Wärmevorrichtung eine Anzahl von n Elektroheizelementen (2) aufweist, wobei diese derart angesteuert sind, dass 2ⁿ - 1 verschiedene Leistungsstufen implementiert sind.

9. Elektrische Wärmevorrichtung nach einem der vorhergehenden Ansprüche, **dadurch gekennzeichnet, dass** die elektrische Wärmevorrichtung ein Ausgleichselement mit einer hohen Wärmekapazität aufweist, das über das Elektroheizelement (2) aufheizbar ist und kontinuierlich zur Harmonisierung der Wärmeabgabe der elektrischen Wärmevorrichtung Wärme, insbesondere in die dem Elektroheizelement abgewandte Richtung, abzugeben vermag.

10. Elektrische Wärmevorrichtung nach dem vorhergehenden Anspruch, **dadurch gekennzeichnet, dass** das Ausgleichselement mantel- oder schlauchartig um das Elektroheizelemente (2) herum angeordnet ist.

11. Elektrische Wärmevorrichtung nach dem vorhergehenden Anspruch, **dadurch gekennzeichnet, dass** das Ausgleichselement ein metallisches Gewebe oder eine metallische Folie aufweist.

12. Elektrische Wärmevorrichtung nach Anspruch 9, **dadurch gekennzeichnet, dass** das Ausgleichselement ein Kissen oder ein doppelwandiger Schlauch mit einem darin angeordneten Gel ist, das eine hohe Wärmekapazität aufweist.
